(19) Europäisches Patentamt
European Patent Office
Office européen des brevets

(11) Veröffentlichungsnummer: **0 405 254 A2**

(12) **EUROPÄISCHE PATENTANMELDUNG**

(21) Anmeldenummer: **90111335.7**

(51) Int. Cl.5: **C07D 223/22**, A61K 31/55

(22) Anmeldetag: **15.06.90**

(30) Priorität: **30.06.89 DD 330174**

(43) Veröffentlichungstag der Anmeldung: **02.01.91 Patentblatt 91/01**

(84) Benannte Vertragsstaaten: **AT CH DE ES FR GB IT LI SE**

(71) Anmelder: **VEB Arzneimittelwerk Dresden**
**Wilhelm-Pieck-Strasse 35 Postfach 89/90**
**DDR-8122 Radebeul 1(DD)**

(72) Erfinder: **Wunderlich, Helmut, Dr.**
**Westendring 22**
**DD-8027 Dresden(DD)**
Erfinder: **Stark, Andreas, Dr.**
**Robert-Koch-Strasse 12**
**DD-8122 Radebeul(DD)**
Erfinder: **Zenker, Lothar, Dr.**
**Sachsenstrasse 27**
**DD-8122 Radebeul(DD)**
Erfinder: **Lohmann, Dieter, Dr.**
**Hoflössnitzstrasse 30**
**DD-8122 Radebeul(DD)**
Erfinder: **Poppe, Hildegard, Dr.**
**Kieler Strasse 6**
**DD-8080 Dresden(DD)**
Erfinder: **Bartsch, Reni, Dr.**
**Stephensonstrasse 11**
**DD-8045 Dresden(DD)**
Erfinder: **Skoldinov, Aleksandr Petrovic, Prof.,Dr.**
**Casovaja Strasse 25, Korpus 2, Wohnung 99**
**Moskau 125315(SU)**
Erfinder: **Kaverina, Natalja Veniaminovna, Prof. Dr.**
**W. Ulbricht Strasse 3, Wohnung 32**
**Moskau 125057(SU)**
Erfinder: **Grizenko, Anna Nikitiona, Dr.**
**Jenisseijskaja Strasse 28, Korpus 2, Wohnung 213**
**Moskau 129281(SU)**
Erfinder: **Lyskovzev, Valentin Viktorovic, Dr.**
**M. Molcanovka Strasse 6, Wohnung 1**
**Moskau G-69(SU)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

(54) **3-Carbalkoxyamino-5-(alpha-aminopropionyl)-5H-dibenz(b,f)-azepine und ihre Herstellung und Verwendung.**

(57) Die Erfindung betrifft neue 3-Carbalkoxyamino-5-(alpha-aminopropionyl)-5H-dibenz[b,f]azepine der allgemeinen Formel I,

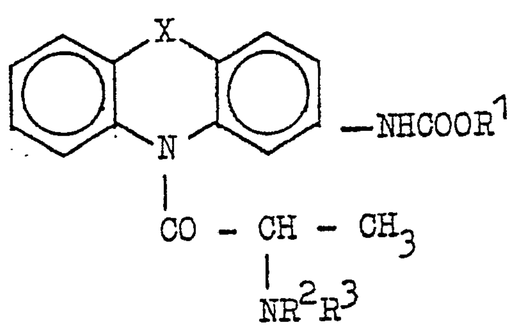
(I),

EP 0 405 254 A2

worin bedeuten:
$R^1$ geradkettiges oder verzweigtes $C_{1-3}$-Alkyl,
$R^2$ und $R^3$, zugleich oder unabhängig Wasserstoff, $C_{1-6}$-Alkyl, das geradkettig, verzweigt oder cyclisch sein kann, oder $\beta$-Oxethyl oder
$R^2$ und $R^3$ zusammen mit dem N-Atom, an das sie gebunden sind, einen 5- oder 6-gliedrigen heterocyclischen Ring, wie Morpholin oder Piperidin,
und
X $-CH_2-CH_2-$ oder $-CH=CH-$,
und ihre Salze, insbesondere die physiologisch verträglichen Salze, Verfahren zu ihrer Herstellung und Zwischenprodukte sowie ihre pharmakologische Verwendung insbesondere als Antiarrhythmika.

Zur Herstellung dieser Verbindungen werden zunächst aus 3-Carbalkoxyamino-5H-dibenz b,f azepinen durch Umsetzung mit alpha-Halogenpropionylhalogeniden bisher nicht bekannte 3-Carbalkoxyamino-5-(alpha-halogenpropionyl)-5H-dibenz[b,f]azepine erhalten. Durch deren Umsetzung mit Ammoniak oder primären oder sekundären Aminen werden die Verbindungen der Formel I gewonnen, die gegebenenfalls in ihre Salze übergeführt werden.

# 3-CARBALKOXYAMINO-5-(ALPHA-AMINOPROPIONYL)-5H-DIBENZ [B,F] -AZEPINE UND IHRE HERSTELLUNG UND VERWENDUNG

Die Erfindung betrifft neue 3-Carbalkoxyamino-5-(alpha-aminopropionyl)-5H-dibenz [b,f] azepine der allgemeinen Formel I,

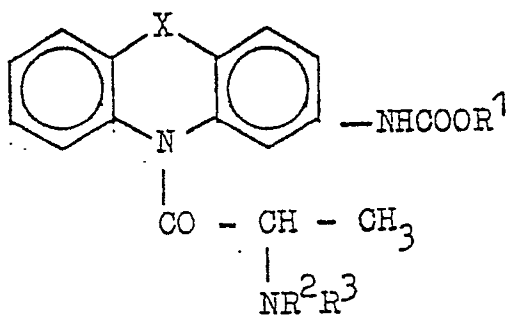

(I),

worin bedeuten:

$R^1$ einen geradkettigen oder verzweigten Alkylrest mit 1 bis 3 C-Atomen,

$R^2$ und $R^3$, die gleich oder verschieden sein können, jeweils Wasserstoff, einen Alkylrest mit 1 bis 6 C-Atomen, der geradkettig, verzweigt oder ringgeschlossen sein kann, oder einen β-Oxethylrest oder

$R^2$ und $R^3$ zusammen mit einem N-Atom, an das sie gebunden sind, einen 5- oder 6-gliedigen heterocyclischen Ring, wie Morpholin oder Piperidin,

und

X $-CH_2-CH_2-$ oder $-CH=CH-$,

und ihre Salze, insbesondere mit physiologisch verträglichen anorganischen oder organischen Säuren, Verfahren und Zwischenprodukte zu ihrer Herstellung sowie die Verwendung dieser Verbindungen als Wirkstoffe in pharmazeutischen Mitteln, insbesondere mit antiarrhythmischer Wirksamkeit. Die Verbindungen stellen potentielle Pharmaka dar, die aufgrund ihrer antiarrhythmischen Wirkungen in der Therapie von Herzrhythmusstörungen vorteilhaft eingesetzt werden können.

Die Verbindungen der allgemeinen Formel I sind neu, da weder ihre Herstellung noch ihre pharmakologischen Eigenschaften vorbeschrieben sind. Gegenüber bereits bekannten Verbindungen mit antiarrhythmischer Aktivität zeigen die erfindungsgemäßen Verbindungen entscheidende strukturelle Unterschiede.

Es sind bereits zahlreiche Stoffe bekannt, die in der Therapie von Herzrhythmusstörungen angewendet werden. Hierzu rechnen u.a. Chinidin, Lidocain, Procainamid, Mexiletin, Disopyramid, Propranolol und Verapamil. Viele der bekannten Antiarrhythmika zeigen unerwünschte Nebenwirkungen sowie teilweise ungenügende Wirksamkeit bei bestimmten Arrhythmieformen. Besonders für die Langzeitkontrolle von Herzrhythmusstörungen ist keines der bekannten Mittel vollständig zufriedenstellend.

Die stark unterschiedlichen chemischen Strukturen dieser Stoffe lassen keine allgemeingültigen Regeln zur Beschreibung von Struktur-Wirkungs-Beziehungen erkennen. Insbesondere zeigen die genannten Therapeutika keine erkennbaren Strukturzusammenhänge mit den erfindungsgemäßen Verbindungen der allgemeinen Formel I.

In DE-A-22 35 745 und DE-A-24 00 450 ist die Darstellung von Analogen des Lidocains beschrieben, wobei besonders das 2-Amino-2′,6′-propionoxylidid (Tocainid) genannt wird, welches im Gegensatz zu Lidocain auch oral verabreicht werden kann. Diese primären Acylanilide sind hinsichtlich der Struktur von den erfindungsgemäßen Verbindungen verschieden.

In SU-A-332 835 sind verschiedene 10-β-Dialkylaminopropionyl-2-carbalkoxyamino-phenothiazine beschrieben, für die antiarrhythmische, spasmolytische und weitere pharmakologische Wirkungen nachgewiesen worden sind. Präparate aus dieser Reihe, wie Morazicin oder Ethazicin, haben Eingang in die Humanmedizin gefunden. Ein Kennzeichen dieser Gruppe von Verbindungen ist der zur Oxidation neigende Phenothiazinring. Durch Oxidation des Ringschwefelatoms entstehen aus diesen Verbindungen bei der Lagerung und im Verlaufe des biologischen Abbaus die entsprechenden 5-Sulfoxide, wobei Wirkungsabschwächung bis zum Wirkungsverlust eintritt. Für die medizinische Anwendung leitet sich daraus die Notwendigkeit ab, die Applikation von Arzneiformen dieser Stoffe in relativ kurzen Zeitabständen zu wiederholen, um die erforderlichen Blutspiegelkonzentrationen zu gewährleisten.

Zusätzlich führt die für die genannten Therapeutika kennzeichnende β-Dialkylaminopropionyl-Gruppe in

10-Stellung zu einer weiteren Begrenzung hinsichtlich der chemischen Stabilität, weil sich mit dieser Substitution eine dem Fachmann hinreichend bekannte β-Eliminierung der Dialkylaminogruppe verbindet.

Die β-Eliminierung tritt auch als Nachteil bei den 3-Carbalkoxyamino-5-(β-dialkylaminopropionyl-10, 11-dihydro-5H-dibenz [b,f]azepinen auf, die in DD-A-152 782 sowie SU-A-1 089 089 beschrieben sind.

Gegenüber den in den letztgenannten Druckschriften ferner aufgeführten 3-Carbalkoxyamino-5-dialkylaminoacetyl-10,11-dihydro-5H-dibenz [b,f]azepinen weisen die erfindungsgemäßen Verbindungen in verschiedener Hinsicht günstigere pharmakologische Effekte auf. Wirkungsstärke, Wirkungsdauer, therapeutische Breite und Resorptionsverhalten grenzen die neuen Verbindungen in vorteilhafter Weise von bekannten Stoffen der genannten Literatur ab.

Der Erfindung liegt die Aufgabe zugrunde, neue 5-substituierte 3-Carbalkoxyamino-5H-dibenz [b,f]-azepine mit antiarrhythmischer Wirkung für die Anwendung in der Humanmedizin, Verfahren zu ihrer Herstellung sowie entsprechende pharmazeutische Mittel anzugeben.

Die Aufgabe wird anspruchsgemäß gelöst. Die abhängigen Ansprüche betreffen vorteilhafte Ausführungsformen der Erfindung.

Die erfindungsgemäßen Verbindungen sind auf einfache Weise herstellbar, indem als Ausgangsstoffe an sich bekannte 3-Carbalkoxyamino-5H-dibenz [b,f]azepine eingesetzt werden. Das erfindungsgemäße Verfahren zur Herstellung der 3-Carbalkoxyamino-5-(alpha-aminopropionyl)-5H-dibenz [b,f]azepine der allgemeinen Formel I wie oben definiert ist gekennzeichnet durch

(A) Umsetzung eines 3-Carbalkoxyamino-5H-dibenz [b,f]azepins der allgemeinen Formel II

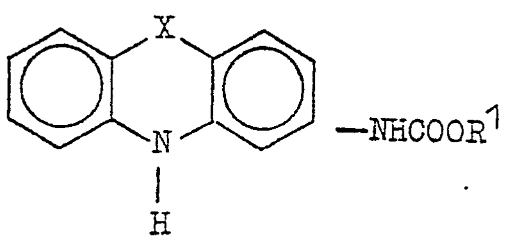

(II)

mit $R^1$ wie oben

mit einem alpha-Halogenpropionylhalogenid der allgemeinen Formel III,

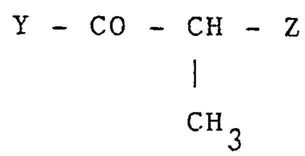

(III),

in der Y und Z Chlor oder Brom bedeuten,

zu einer Verbindung der allgemeinen Formel IV

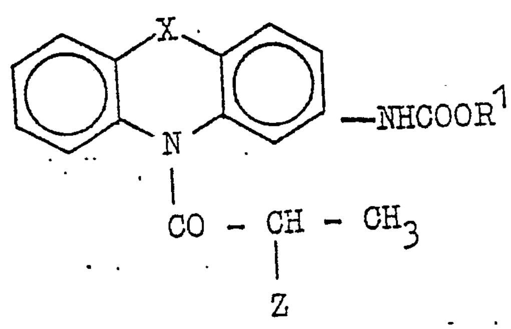

(IV)

mit $R^1$, X und Z wie oben

und

(B) Umsetzung der Verbindung der allgemeinen Formel IV mit einem Amin der allgemeinen Formel V

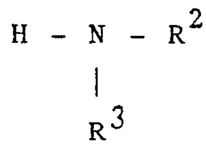

(V)

mit $R^2$ und $R^3$ wie oben
zur entsprechenden Verbindung der Formel I
sowie gegebenenfalls
(C) Überführung der erhaltenen Verbindungen der Formel I mit anorganischen oder organischen Säuren in entsprechende Salze
oder gegebenenfalls Überführung von Salzen von Verbindungen der allgemeinen Formel I in die entsprechenden freien Basen.

Die zur Herstellung der Verbindungen der allgemeinen Formel IV erforderlichen Ausgangsstoffe der allgemeinen Formeln II und III sind bekannt. Bei ihrer Umsetzung entstehen Verbindungen der allgemeinen Formel IV, die bisher nicht beschrieben sind. Sie sind ebenfalls Gegenstand der Erfindung.

So wird. z.B. aus alpha-Brompropionylchlorid und 3-Carbethoxyamino-5H-dibenz [b,f]azepin in einem inerten Lösungs mittel, wie Benzol, Toluol, Xylol, Chlorbenzol, Chloroform oder Dichlorethan, unter Abspaltung von Chlorwasserstoff 3-Carbethoxyamino-5-(alpha-brompropionyl)-5H-dibenz [b,f]azepin erhalten. Die Reaktion kann auch in Gegenwart von Halogenwasserstoff-Acceptoren, wie Pyridin, Triethylamin, Natrium- oder Kaliumcarbonat, durchgeführt werden.

Erfindungsgemäß werden die Verbindungen der allgemeinen Formel IV mit einem Amin der allgemeinen Formel V zu Verbindungen der allgemeinen Formel I umgesetzt. Die dafür erforderlichen Amine der allgemeinen Formel V, wie z.B. Ammoniak, Methyl-, Ethyl-, Propyl-, Dimethyl- oder Diethylamin, Morpholin oder Piperidin, sind technisch leicht zugängliche Produkte. Die Umsetzung der Verbindungen der allgemeinen Formel IV mit dem Amin der allgemeinen Formel V wird vorzugsweise in Lösungsmitteln, wie Benzol, Toluol, Chlorbenzol, Xylol, Chloroform oder niederen Alkoholen, bei Temperaturen bis zur Siedetemperatur des Lösungsmittels durchgeführt. Es ist auch möglich und teilweise von Vorteil, in Gegenwart von Wasser zu arbeiten. In diesem Falle ist die Verwendung handelsüblicher wäßriger Lösungen der entsprechenden aminischen Reaktionspartner, wie wäßrige Ammoniak-, Methylamin- oder Ethylaminlösungen, zweckmäßig.

Insbesondere bei der Anwendung von leicht flüchtigen Reaktionspartnern der allgemeinen Formel V kann es für einen optimalen Reaktionsverlauf von Vorteil sein, wenn man die Reaktion unter erhöhtem Druck ausführt.

Man kann die Reaktion zwischen der Verbindung der allgemeinen Formel IV und dem Amin der allgemeinen Formel V auch in Gegenwart von Halogenwasserstoff-Acceptoren durchführen. Als solche eignen sich z.B. Kaliumcarbonat, Natriumcarbonat, tertiäre Amine und andere, gegebenenfalls in wäßriger Lösung. Vorteilhaft ist es, wenn man als Halogenwasserstoff-Acceptor einen Überschuß des für die Reaktion eingesetzten Amins der allgemeinen Formel V einsetzt, wobei vorzugsweise Aminmengen zwischen 100 bis 800 Stoffmengenanteilen in % zur Anwendung kommen sollten. Dies wirkt sich insbesondere günstig auf die weitgehende Unterdrückung einer möglichen Nebenreaktion aus, die darin besteht, daß eine Verbindung der allgemeinen Formel I, in der $R^3$ Wasserstoff ist, mit einem weiteren Molekül Alkylhalogenid der allgemeinen Formel IV zu einem Nebenprodukt der allgemeinen Formel VI

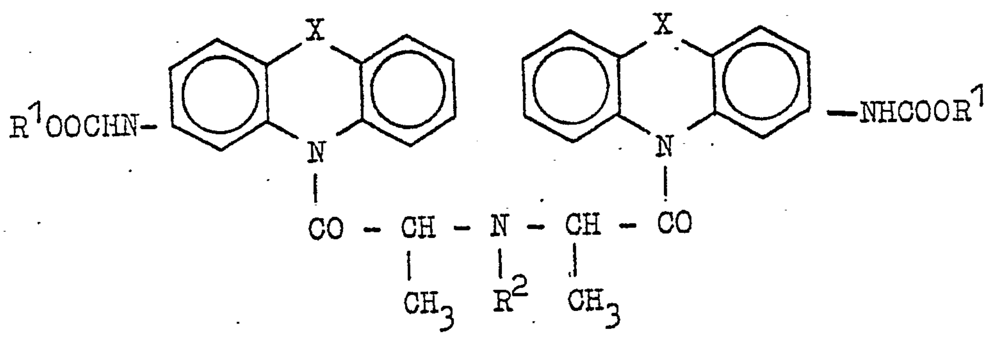

(VI)

reagieren kann, in der $R^1$, $R^2$ und X die gleiche Bedeutung wie oben besitzen.

Die erfindungsgemäßen Verbindungen der allgemeinen Formel I stellen ölige oder kristalline Basen dar,

die gegebenenfalls hydratisiert sind. In an sich bekannter Weise können sie durch Umsetzung mit physiologisch verträglichen anorganischen oder organischen Säuren, wie Salzsäure, Schwefelsäure, Weinsäure, Zitronensäure u.a., in ihre Salze, die gegebenenfalls hydratisiert sein können, übergeführt werden. Für die galenische Verarbeitung der erfindungsgemäßen Verbindungen der allgemeinen Formel I verwendet man zweckmäßigerweise ihre physiologisch verträglichen Salze. Aus den Salzen lassen sich in an sich bekannter Weise, z.B. durch Einwirkung alkalischer Reagentien, die erfindungsgemäßen Basen der allgemeinen Formel I zurückgewinnen.

Durch die Erfindung werden neue, hocheffektive Herz-Kreislauf-Pharmaka zur Verfügung gestellt, die aufgrund ihrer ausgeprägten antiarrhythmischen Wirkung in der Therapie von Herzrhythmusstörungen verwendet werden können. Insbesondere umfaßt die Erfindung solche Stoffe, die sich durch ihre Wirkungsstärke, verbesserte therapeutische Breite, günstigeres Resorptionsverhalten und erhöhte Stabilität im Vergleich zu bekannten Antiarrhythmika auszeichnen. Nachteile des Standes der Technik, wie z.B. die Sulfoxidation phenothiazinhaltiger Antiarrhythmika oder die $\beta$-Eliminierung der Amingruppe bei bestimmten antiarrhythmisch wirksamen Stoffklassen, werden durch die Erfindung vermieden.

Die Resultate der tierexperimentellen Untersuchungen zur pharmakologischen Aktivität werden im folgenden näher dargestellt.

Die erfindungsgemäßen 5H-Dibenz [b,f]azepine der allgemeinen Formel I zeichnen sich bei der tierexperimentellen Untersuchung durch eine ausgeprägte antiarrhythmische Wirkung aus. In verschiedenen pharmakologischen Modellen zur Prüfung der antiarrhythmischen Wirksamkeit erweisen sich die erfindungsgemäßen Verbindungen als wesentlich stärker wirksam als zum Vergleich herangezogene, in die Therapie von Herzrhythmusstörungen eingeführte Therapeutika, wie Lidocain, Mexiletin und Moracizin. Insbesondere zeigen die erfindungsgemäßen Verbindungen auch gegenüber den in DD-A-152 782 und SU-A-1 089 089 beschriebenen strukturverwandten 10,11-Dihydro-5H-dibenz [b,f]azepinen, von denen das 3-Carbethoxyamino-5-dimethylaminoacetyl- 10,11-dihydro--5H-dibenz [bf]azepin sich in der klinischen Prüfung befindet, vorteilhafte Effekte.

In der nachstehenden Tabelle sind für typische erfindungsgemäße Verbindungen pharmakologische Daten ($ED_{73\ i.v.}$/ $ED_{73\ p.o.}$; $Q_1 = ED_{73\ p.o.}/ED_{73\ i.v.}$; $LD_{50\ i.v.}$; $Q_2 = LD_{50\ i.v.}/ED_{73\ i.v.}$) aufgeführt.

Da sich bei etwa gleicher Verträglichkeit mit der erhöhten Wirkungsstärke eine am Aconitin-Modell der Ratte im Einzelfall sogar wesentliche Verbesserung der therapeutischen Breite ergibt (siehe die Tabelle, $Q_2 = LD_{50\ i.v.}/ ED_{73\ i.v.}$), bieten sich die erfindungsgemäßen Verbindungen mit Vorzug für eine medizinische Verwendung bei der Behandlung von Herzrhythmusstörungen an.

Hinsichtlich ihrer Verträglichkeit ist besonders hervorzuheben, daß trotz einer den Psychopharmaka verwandten trizyklischen Grundstruktur weder sedierende noch zentral erregende oder andere psychisch ungünstige Wirkungen nachweisbar sind.

Hervorzuheben ist weiterhin, daß die erfindungsgemäßen Verbindungen der allgemeinen Formel I eine im Einzelfall sogar wesentlich verbesserte Resorption aufweisen (siehe die Tabelle, $Q_1 = ED_{73\ p.o.}/ED_{73\ i.v.}$). Bei einem möglichst kleinen Resorptionsquotienten $Q_1$ reduziert sich die Substanzmenge erheblich, die bei oraler Anwendung der Arzneistoffe in den Tabletten enthalten sein muß.

Für die Anwendung der erfindinngsgemäßen Verbindungen in der Therapie von Herzrhythmusstörungen eignen sich verschiedenartige galenische Formulierungen, wie Tabletten, Dragees, Tropfenlösungen oder Injektionslösungen und an dere Zubereitungsformen. In Abhängigkeit vom Schweregrad der Herzrhthmusstörung kann die ein- oder mehrmalig täglich oral zu applizierende Dosis in einem mittleren Dosisbereich von 5 bis 100 mg liegen.

| Nr. | X | $R^1$ | $R^2$ | $R^3$ | Aconitin (Ratte) $ED_{73}$ (mg/kg) iv. | p.o. | $Q_1$ | akute Toxizität (Ratte) $LD_{50}$ (mg/kg) iv. | $Q_2$ |
|---|---|---|---|---|---|---|---|---|---|
| 1 | $-CH_2-CH_2-$ | $C_2H_5$ | $CH_3$ | $CH_3$ | 2,4 (1,5-3,8) | 24 (16-36) | 10 | 20 (17-24) | 8 |
| 2 | $-CH_2-CH_2-$ | $C_2H_5$ | $C_2H_5$ | $C_2H_5$ | 1,3 (0,95-1,8) | 11 (9-13) | 9 | 12 (11-13) | 9 |
| 3 | $-CH_2-CH_2-$ | $C_2H_5$ | 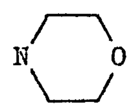 | | 47 (22-103) | bis 80 – | – | VP > 70 | ca. 2 |
| 4 | $-CH_2-CH_2-$ | $C_2H_5$ | $CH_3$ | H | 0,12 (0,062-0,22) | 19 (10-34) | 158 | 12 (11-14) | 100 |
| 5 | $-CH_2-CH_2-$ | $C_2H_5$ | $C_2H_5$ | H | 0,16 (0,10-0,25) | 7 (6-8) | 44 | 6,3 (5,7-7,0) | 39 |
| 6 | $-CH_2-CH_2-$ | $C_3H_7$(iso) | $CH_3$ | H | 0,55 (0,20-1,5) | 12 (6,4-22) | 22 | 21 (19-21) | 38 |
| 7 | $-CH_2-CH_2-$ | $C_3H_7$(iso) | $C_2H_5$ | H | 0,33 (0,18-0,61) | 20 (12-31) | 61 | 10 (8,8-12) | 30 |

Fortsetzung der Tabelle

| | | | | | | | | | |
|---|---|---|---|---|---|---|---|---|---|
| 8 | $-CH_2-CH_2-$ | $CH_3$ | $CH_3$ | H | 0,86 (0,49-1,5) | 14 (8,9-23) | 16 | 24 (22-25) | 28 |
| 9 | $-CH_2-CH_2-$ | $CH_3$ | $CH_2-CH_2-OH$ | H | 4,0 (2,4-6,9) | - | - | 55 (52-57) | 14 |
| 10 | -CH=CH- | $C_2H_5$ | $CH_3$ | H | 0,75 (0,43-1,3) | 4,9 (3,0-8,1) | 6,5 | 14 (13-15) | 19 |
| 11 | -CH=CH- | $C_2H_5$ | $C_2H_5$ | H | 0,80 (0,29-2,2) | 14 (7-27) | 18 | 14 (13-15) | 18 |
| 12 | $-CH_2-CH_2-$ | $C_2H_5$ | $C_3H_7$(n) | H | 0,16 (0,058-0,43) | 3,5 (2,2-5,8) | 22 | 2,5 (2,2-2,8) | 16 |
| 13 | $-CH_2-CH_2-$ | $C_2H_5$ | H | H | 0,31 (0,21-0,45) | 14 (11-17) | 45 | 21 (19-23) | 68 |
| | Lidocain | | | | 9,2 (5,9-15) | - | - | 18 (17-21) | 2,0 |
| | Moracizin | | | | 1,1 (0,41-3,1) | 112 (28-445) | 102 | 11 (9,6 - 12) | 10 |

Fortsetzung der Tabelle

| | | | | | |
|---|---|---|---|---|---|
| Bonnecor(R) | 0,28 (0,13-0,62) | 5,7 (1,6-20) | 20 | 11 (10-12) | 39 |
| Mexiletin | 15 (8,8-25) | 91 *) (58-143) | - | 41 (34-50) | 2,7 |

## Legende zur Tabelle

$ED_{73}$ i.v. und p.o. = Dosis in mg/kg, welche die Aconitinarrhythmie an weiblichen Wistarratten nach i.v.- bzw. p.o.-Applikation der Testsubstanz bis zum Auftreten vereinzelter ventrikulärer Extrasystolen hemmt. Durchführung wie bei Femmer, K., und Mitarb., Pharmazie 31 , S. 36, 1976. Die Testsubstanzen werden bei i.v.-Gabe 2 min und bei oraler Gabe 60 min vor dem Aconitin verabfolgt. Berechnung nach der Probit-Regressionsanalyse mit Vertrauensbereich p = 0,05 (Werte in Klammern)

$LD_{50\ i.v.}$ = Letale Dosis in mg/kg bei 50 % der Versuchstiere einer orientierenden akuten Toxizität an männlichen oder weiblichen Wistarratten hauseigener Zucht;
i.v.-Applikation in die Schwanzvene, 3 - 4 Dosierungsgruppen zu je 10 Tieren. Berechnung nach der Probit-Regressionsanalyse mit Vertrauensbereich p = 0,05 (Werte in Klammern)

$$Q_1 = \text{Quotient } \frac{ED_{73}\ \text{p.o.}}{ED_{73}\ \text{i.v.}}$$ zur Ermittlung der Resorption

$$Q_2 = \text{Quotient } \frac{LD_{50}\ \text{i.v.}}{ED_{73}\ \text{i.v.}}$$ zur Ermittlung der therapeutischen Breite

Werte mit Stern *) $ED_{50}$ i.v. mit Vertrauensgrenzen

Ausführungsbeispiele

Beispiel 1:

20,9 g (0,05 mol) 5-alpha-Brompropionyl-3-carbethoxyamino-10,11-dihydro-5H-dibenz [b,f]azepin werden in 150 ml 96 %igem Ethanol suspendiert. Man setzt 30 ml ca. 35%ige wäßrige Methylaminlösung zu, rührt die Mischung 16 Stunden bei 50 -70 °C und läßt über Nacht bei Raumtemperatur stehen, wobei ein feinkristalliner Niederschlag ausfällt. Dieser wird abgesaugt, zweimal mit je 25 Ethanol gewaschen und bei 100 °C getrocknet. Man erhält 15 g 3-Carbethoxyamino-5-(alpha-methylaminopropionyl)-10,11-dihydro-5H-dibenz [b,f]azepin, das durch Ausrühren in Aceton und Umkristallisation aus n-Butanol weiter gereinigt werden kann und dann bei 201 - 203 °C schmilzt.
10 g der freien Base werden in 40 ml Methylenchlorid gelöst,dann wird mit gasförmiger Salzsäure auf pH 2 gestellt und das gebildete Hydrochlorid durch Zugabe von Ethylether ausgefällt und abgesaugt. Man erhält 3-Carbethoxyamino-5-(alpha-methylamino-propionyl)-10,11-dihydro-5H-dibenz [b,f]azepin-hydrochlorid von Fp. 168 °C.

Das Ausgangsmaterial kann beispielsweise folgendermaßen hergestellt werden:
28,2 g (0,1 mol.) 3-Carbethoxyamino-10,11-dihydro-5H-dibenz, [b,f]azepin werden mit 300 ml Toluol und 20 g (0,11 mol) 2-Brompropionylchlorid vermischt und im siedenden Wasserbad 4 Stunden gerührt, wobei HCl-Gas entweicht. Man läßt auf Raumtemperatur abkühlen, filtriert und dampft unter vermindertem Druck zur

Trockne ein. Bei Zusatz von Diethylether tritt nach vorübergehender Lösung Kristallisation ein. Der Niederschlag wird abgesaugt, mit Ether gewaschen und getrocknet. Man erhält 39 g 3-Carbethoxyamino-5-(alphabrompropionyl)-10,11-dihydro-5H-dibenz [b,f]azepin, das zur Reinigung aus dar fünffachen Menge Isopropanol umkristallisiert wird.

In analoger Weise kann man ferner 3-Carbethoxyamino-5-(alpha-ethylaminopropionyl)-10,11-dihydro-5H-dibenz [b,f]azepin-hydrochlorid, Fp. 172 °C und 3-Carbethoxyamino-5-(alpha-propylaminopropionyl)-10,11-dihydro-5H-dibenz [b,f]azepin-hydrochlorid, Fp. 177 - 178 °C, herstellen.

Beispiel 2:

18,6 g (0,05 mol) 3-Carbethoxyamino-5-(alpha-chlorpropionyl)-10,11-dihydro-5H-dibenz [b,f]azepin werden in einem Gemisch aus 200 ml wäßriger Ammoniaklösung und 250 ml 96 %igem Ethanol, das bei Raumtemperatur mit gasförmigem Ammoniak gesättigt wurde, suspendiert und im Laborautoklaven 18 Stunden bei 60 -65 °C gerührt. Anschließend wird unter vermindertem Druck bei 70 °C das Lösungsmittel abgetrieben. Der viskose Rückstand wird in verdünnter Salzsäure gelöst und die Lösung mit Aktivkohle behandelt. Durch Zusatz von konz. Ammoniaklösung wird alkalisch gestellt und die so freigesetzte Base mit 100 ml Methylenchloride extrahiert. Nach Trocknen über Natriumsulfat wird mit etherischer Salzsäure das Hydrochlorid gefällt. Man erhält 3-Carbethoxyamino-5-(alpha-aminopropionyl)-10,11-dihydro-5H-dibenz [b,f]-azepin-hydrochlorid vom Fp. 193 - 195 °C.
Das Ausgangsmaterial kann man beispielsweise analog der Vorgehensweise im Beispiel 1 herstellen. Man erhält 3-Carbethoxy-amino-5-(alpha-chlorpropionyl)-10,11-dihydro-5H-dibenz-[b,f]azepin vom Fp. 180 - 181 °C.

Folgende Verbindung wurde z. B. analog dargestellt: 3-Carbisopropoxyamino-5-(alpha-methylaminopropionyl)-10,11-dihydro-5H-dibenz [b,f]azepin, Fp. 209 - 211 °C (Ethanol).

Beispiel 3:

10 g (0,024 mol) 3-Carbethoxyamino-5-(alpha-brompropionyl)-5H-dibenz [b,f] azepin werden im Laborautoklaven mit 15 ml ca. 35 %iger wäßriger Methylaminlösung und 75 ml Ethanol versetzt und 12 Stunden auf 90 - 95 °C erwärmt. Nach Abkühlen auf 4 °C wird der entstandene Niederschlag abgesaugt, mit 50 ml Aceton verrieben, wiederum abesaugt und getrocknet. Man erhält 7 g 3-Carbethoxyamino-5-(alpha-methylaminopropionyl)-5H-dibenz [b,f] azepin, das durch Lösen in verdünnter Salzsäure und Fällen mit konz. Ammoniaklösung gereinigt werden kann und dann bei 217 - 219 °C schmilzt.
Das Ausgangsmaterial kann beispielsweise folgendermaßen hergestellt werden:
28 g (0,1 mol) 3-Carbethoxyamino-5H-dibenz [b,f] azepin werden in 300 ml Benzol und 20g (0,11 mol) 2-Brompropionylchlorid bei der Siedetemperatur des Lösungsmittels gerührto Die Reaktion ist nach etwa 3 Stunden beendet. Man dampft unter vermindertem Druck zur Trockne ein und wäscht den viskosen Rückstand mit Wasser. Durch Zusatz von Ethylether setzt Kristallisation ein. Man saugt ab und kristallisiert aus der dreifachen Menge Isopropanol um. Man erhält 35 g 3-Carbethoxyamino-5-(alpha-brompropionyl)-5H-dibenz [b,f]azepin vom Fp. 163 - 165 °C.

Beispiel 4:

In analoger Weise wie im Beispiel 3 beschrieben kann man ferner 3-Carbethoxyamino-5-(alpha-ethylaminopropionyl)-5H-dibenz [b,f] azepin herstellen. 6 g der freien Base werden in Ethylether gelöst und durch Zusatz von isopropanolischer Salzsäure sauer gestellt. Das gebildete Hydrochlorid wird beim Verreiben mit frischem Ethylether kristallin.
Man erhält 3-Carbethoxyamino-5-(alpha-ethylaminopropionyl)-5H-dibenz [b,f] azepinhydrochlorid vom Fp. 206 - 209 °C.

Beispiel 5:

4,0 g (0,01 mol) 3-Carbomethoxyamino-5-(alpha-brompropionyl)-10,11-dihydro-5H-dibenz [b,f] azepin, 3,3 g (0,055 mol) 2-Aminoethanol und 70 ml Toluol rührt man 6 Stunden bei 100 - 110 °C. Man dampft

unter vermindertem Druck zur Trockne ein, wäscht den Rückstand mit Wasser und löst ihn in verdünnter Salzsäure, reinigt die Lösung mit Aktivkohle und scheidet die Base durch Alkalisieren mit verdünnter Natronlauge ab. Man dekantiert, löst den Rückstand in Aceton und fällt durch Zusatz einer etherischen Chlorwasserstofflösung das Hydrochlorid. Man erhält 3-Carbomethoxyamino-5-(alpha-hydroxyethylamino-propionyl)-10,11-dihydro-5H-dibenz [b,f] azepin-hydrochlorid vom Fp. 163 - 164 °C (z).
Das Ausgangsmaterial kann z. B. wie folgt hergestellt werden:
13,5 g (0,05 Mol) 3-Carbomethoxyamino-10,11-dihydro-5H-dibenz [b,f] azepin, 200 ml Toluol und 12 g (0,06 mol ) 2-Brompropionylchlorid erwärmt man 5 Stunden am Rückfluß, kühlt ab und filtriert den ausgefallenen Niederschlag ab. Nach Umkristallisation aus Toluol erhält man 14 g 3-Carbomethoxyamino-5-(alpha-brompropionyl)-10,11-dihydro-5H-dibenz [b,f] azepin, das bei 158 - 159 °C schmilzt.

**Ansprüche**

1. 3-Carbalkoxyamino-5-(alpha-aminopropionyl)-5H-dibenz-[b,f]azepine der allgemeinen Formel I,

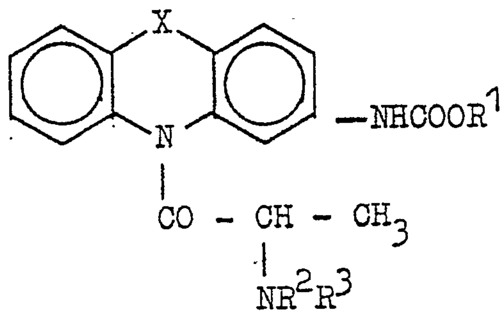
(I),

worin bedeuten:
$R^1$ einen geradkettigen oder verzweigten Alkylrest mit 1 bis 3 C-Atomen,
$R^2$ und $R^3$, die gleich oder verschieden sein können, jeweils Wasserstoff, einen Alkylrest mit 1 bis 6 C-Atomen, der geradkettig, verzweigt oder ringgeschlossen sein kann, oder einen $\beta$-Oxethylrest oder
$R^2$ und $R^3$ zusammen mit dem N-Atom, an das sie gebunden sind, einen 5- oder 6-gliedrigen heterocyclischen Ring, wie Morpholin oder Piperidin, und
X $-CH_2-CH_2-$ oder $-CH=CH-$,
sowie ihre Salze, insbesondere die physiologisch verträglichen Salze.
2. 3-Carbethoxyamino-5-(alpha-methylaminopropionyl)-10,11-dihydro-5H-dibenz[b,f]azepin,
3-Carbethoxyamino-5-(alpha-ethylaminopropionyl)-10,11-dihydro-5H-dibenz[b,f]azepin,
3-Carbethoxyamino-5-(alpha-propylaminopropionyl)-10,11-dihydro-5H-dibenz[b,f]azepin,
3-Carbethoxyamino-5-(alpha-aminopropionyl)-10,11-dihydro-5H-dibenz[b,f]azepin und
3-Carbisopropoxyamino-5-(alpha-methylaminopropionyl)-10,11-dihydro-5H-dibenz[b,f]azepin
sowie die Salze dieser Verbindungen.
3. Verfahren zur Herstellung der 3-Carbalkoxyamino-5-(alpha-aminopropionyl)-5H-dibenz [b,f] azepine der allgemeinen Formel I nach Anspruch 1 oder 2,
gekennzeichnet durch
(A) Umsetzung eines 3-Carbalkoxyamino-5H-dibenz [b,f]azepins der allgemeinen Formel II

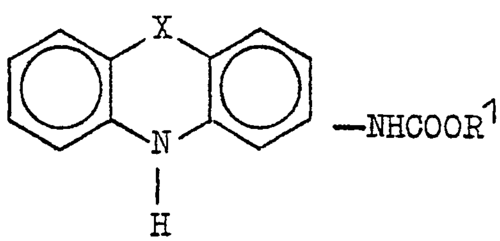
(II)

mit $R^1$ wie in Anspruch 1
mit einem alpha-Halogenpropionylhalogenid der allgemeinen Formel III,

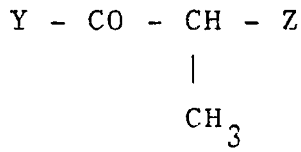 (III),

in der Y und Z Chlor oder Brom bedeuten,
zu einer Verbindung der allgemeinen Formel IV

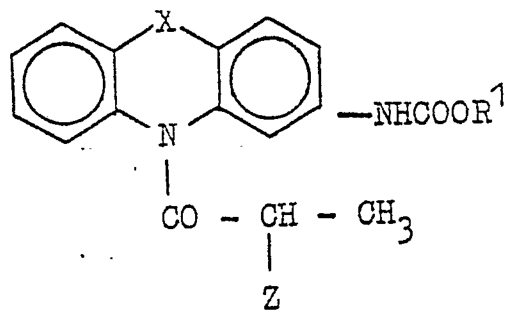 (IV)

mit $R^1$ und X wie in Anspruch 1 und
Z wie oben
und
(B) Umsetzung der Verbindung der allgemeinen Formel IV mit einem Amin der allgemeinen Formel V

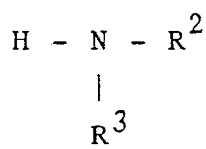 (V)

mit $R^2$ und $R^3$ wie in Anspruch 1
zur entsprechenden Verbindung der Formel I sowie gegebenenfalls
(C) Überführung der erhaltenen Verbindungen der Formel I mit anorganischen oder organischen Säuren in entsprechende Salze
oder gegebenenfalls Überführung von Salzen von Verbindungen der allgemeinen Formel I in die entsprechenden freien Basen.

4. Verfahren nach Anspruch 3, dadurch gekennzeichnet, daß Schritt A in indifferenten organischen Lösungsmitteln, wie Benzol, Toluol, Chlorbenzol, Chloroform oder Dichlorethan, bei Temperaturen bis zur Siedetemperatur des jeweiligen Lösungsmittels durchgeführt wird.

5. Verfahren nach Anspruch 3 oder 4, dadurch gekennzeichnet, daß Schritt B in organischen Lösungsmitteln, wie Benzol, Toluol, Chlorbenzol, Chloroform oder niederen Alkoholen, gegebenenfalls in Gegenwart von Wasser, bei Temperaturen bis zur Siedetemperatur des jeweiligen Lösungsmittels oder Lösungsmittelgemischs durchgeführt wird.

6. Verfahren nach den Ansprüchen 2 und 4, dadurch gekennzeichnet, daß Schritt B unter Druck durchgeführt wird.

7. Verfahren nach den Ansprüchen 2, 4 und 5, dadurch gekennzeichnet, daß Schritt B in Gegenwart von Halogenwasserstoff-Acceptoren, wie Natriumcarbonat oder Kaliumcarbonat, oder tertiären Aminen, wie Triethylamin oder Pyridin, oder mit einem Überschuß an Amin der allgemeinen Formel V durchgeführt wird, der vorzugsweise 100 bis 800 Stoffmengenanteile in % beträgt.

8. Pharmazeutische Mittel, insbesondere zur Behandlung von Arrhythmien des Herz-Kreislauf-Systems, gekennzeichnet durch einen Gehalt an einer oder mehreren Verbindungen der allgemeinen Formel I und pharmazeutisch geeigneten Hilfs- und/oder Trägerstoffen.

9. Verbindungen der allgemeinen Formel IV,

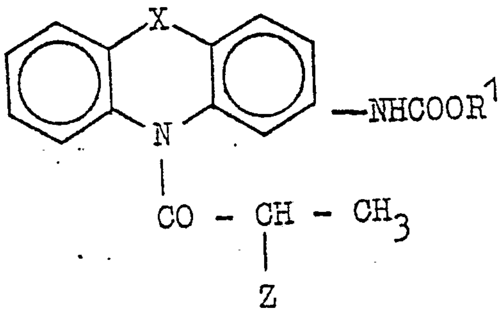

(IV),

in der bedeuten:

$R^1$ einen geradkettigen oder verzweigten Alkylrest mit 1 bis 3 C-Atomen,

X $-CH_2-CH_2-$ oder $-CH=CH-$

und

Z Chlor oder Brom

und ihre Salze mit anorganischen und organischen Säuren.

10. Verwendung der Verbindungen der allgemeinen Formel IV nach Anspruch 9 zur Herstellung von Verbindungen der allgemeinen Formel I nach Anspruch 1 oder 2.